(19) Europäisches Patentamt / European Patent Office / Office européen des brevets

(11) **EP 2 520 631 A1**

(12) **DEMANDE DE BREVET EUROPEEN**

(43) Date de publication:
**07.11.2012 Bulletin 2012/45**

(51) Int Cl.:
*C09K 8/00* (2006.01)  *C12Q 1/64* (2006.01)
*G01N 33/24* (2006.01)

(21) Numéro de dépôt: **12290141.6**

(22) Date de dépôt: **26.04.2012**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**

(30) Priorité: **06.05.2011 FR 1101402**

(71) Demandeur: **IFP Energies Nouvelles
92852 Rueil Malmaison Cedex (FR)**

(72) Inventeurs:
• **Behar, Françoise
  75007 Paris (FR)**
• **Haeseler, Frank
  92250 La Garenne Colombes (FR)**

(54) **Procédé pour prédire les quantités de gaz biogénique générées par transformation biologique de la matière organique**

(57)  - Procédé pour prédire des quantités de gaz biogénique générées par transformation biologique de matière organique déposée dans des sédiments à l'échelle des temps géologiques.

- On considère des motifs structuraux bioréactifs de formules CxHyOvSzNw avec x>6, y>x, v <6, z<6, w<6. Puis, on prélève un échantillon d'une roche de la série sédimentaire à une profondeur proche de la surface, telle que ladite roche n'a subi aucune biodégradation, et l'on détermine des quantités de chaque motif structural bioréactif présent dans cet échantillon. On définit un schéma réactionnel dans lequel les motifs structuraux bioréactifs réagissent parallèlement avec des accepteurs d'électrons, et chacun des motifs structuraux bioréactifs réagit séquentiellement avec ces accepteurs d'électrons. Enfin, on détermine la quantité de gaz biogénique produit par biodégradation des motifs structuraux bioréactifs, au moyen du schéma réactionnel et des quantités de chaque motif structural présent dans l'échantillon.

Fig. 1

## Description

La présente invention concerne le domaine de l'industrie pétrolière, et plus particulièrement le domaine de l'exploration et la production d'énergie fossile issue du sous-sol.

L'invention concerne notamment un procédé pour évaluer les volumes de gaz biogénique généré dans les bassins sédimentaires. Elle fournit ainsi un outil d'évaluation très utile, notamment aux géologues, pour orienter les investigations de nouvelles sources d'énergie fossile, telle que le méthane contenu dans des sédiments peu profonds (entre 0 et 2500 mètres de profondeur).

Un des enjeux stratégiques en exploration pétrolière est de repousser au maximum l'épuisement des ressources en hydrocarbures. Dans ce contexte, des zones jamais explorées compte tenu des coûts de production sont considérés maintenant comme potentiellement exploitables. Parmi ces nouvelles ressources, le gaz biogénique accumulé dans des sédiments peu profonds (moins de 2500 m) est actuellement considéré comme une nouvelle réserve potentielle d'énergie fossile.

A l'inverse des phénomènes de biodégradation des huiles accumulées dans les réservoirs, qui altèrent leur qualité par la destruction des hydrocarbures les plus légers, l'activité microbienne dans les sédiments consomme une partie du carbone contenu dans les sédiments pour le transformer en gaz biogénique qui, en particulier est un mélange $CO_2$ et de méthane dans les conditions de méthanogénèse. Le $CO_2$ pouvant se dissoudre dans l'eau, la proportion de méthane dans la phase gaz peut alors devenir majoritaire.

La biodégradation est un phénomène biologique qui peut affecter la matière organique déposée dans les sédiments. Les bactéries, responsables de ces réactions, sont présentes dans le milieu poreux et vivent dans l'eau qui circule dans le milieu poreux. Ces bactéries utilisent la matière organique présente comme source de carbone et d'énergie pour assurer leur développement et leur maintien. En faisant cela, d'une part elles dégradent sélectivement certains structures de cette matière organique et donc modifient à la fois la quantité et la composition de cette matière organique en place et d'autre part elles génèrent des métabolites comme des espèces gazeuses ($CH_4$, $H_2S$ et $CO_2$) et de nouvelles structures chimiques comme les acides carboxyliques qui à leur tour sont dégradés. Pour assurer le métabolisme bactérien, cette source de carbone et d'énergie ne suffit pas, il faut également que soient présents dans le système des ingrédients comme les accepteurs d'électrons et éventuellement des nutriments. Les réactions biologiques n'ont pas besoin de nutriments comme le phosphore et l'azote ainsi que des métaux; ces éléments sont cependant indispensables à la synthèse des molécules constitutives des bactéries. Leur absence signifie l'arrêt de la croissance bactérienne mais pas celui de leur activité.

Le processus de biodégradation peut être décrit de la façon suivante. Les microorganismes présents dans les milieux poreux utilisent la source d'énergie et de carbone que constituent certaines structures chimiques présentes dans la matière organique avec deux objectifs :

- se développer et produire le plus possible de biomasse tant que l'azote et le phosphore ne sont pas épuisés et que les éléments en trace comme les métaux ne sont pas des facteurs limitants

- se maintenir c'est à dire utiliser l'énergie disponible dans la matière organique à travers des réactions de dégradation qui ne génèrent pas de biomasse supplémentaire. Durant ces réactions, la matière organique sédimentaire est effectivement dégradée et des métabolites sont générés mais aucun accroissement de biomasse n'est observé;

Dans l'industrie pétrolière, il est très important de connaître le rôle de la transformation biologique de la matière organique sédimentaire pour connaître la quantité de gaz biogénique produite à faible profondeur dans un bassin sédimentaire. Pour y parvenir, il faut disposer d'une méthode permettant d'estimer l'effet de la biodégradation sur la quantité et la qualité de la matière organique de départ et par voie de conséquence sur la volumétrie du méthane généré.

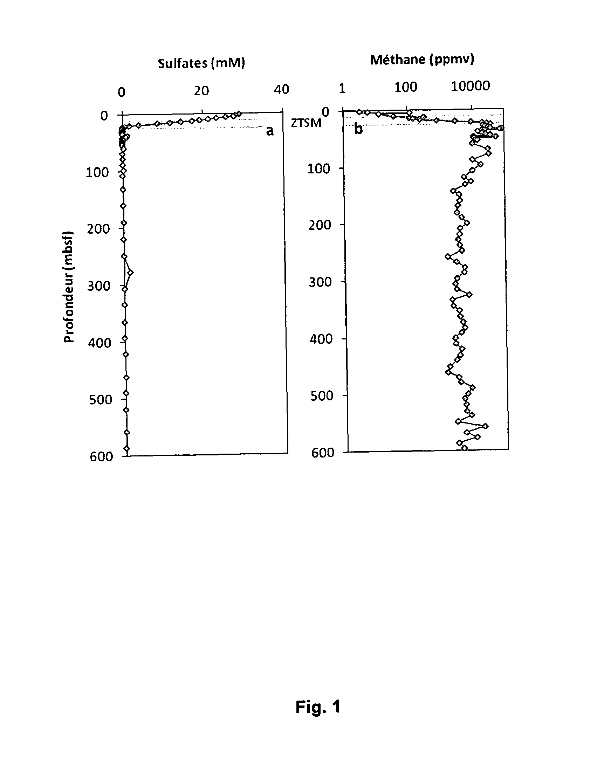

Les réactions microbiennes de transformation de la matière organique sédimentaires sont des réactions successives qui commencent par la dégradation en milieu aérobie avec consommation de l'oxygène (dissous dans l'eau) suivie de la dénitrification dès que les conditions deviennent anoxiques avec consommation des nitrates (également dissous dans l'eau), suivie de la sulfato réduction en condition anaérobie avec consommation des sulfates (qui eux peuvent soit être dissous dans l'eau, soit présents sous la forme de sels précipités dans la roche). La figure 1 sur laquelle sont reportées les teneurs en H2S et CH4 dans une série sédimentaire en fonction de la profondeur illustre tout à fait ces réactions successives.

La dernière réaction est la méthanogénèse en condition anaérobie avec consommation de l'eau présente dans le milieu. Les données disponibles dans la littérature sont principalement des caractérisations moléculaires de gaz dans les campagnes d'échantillonnages et carottages dans différentes zones du globe. La matière organique qui est présente presque essentiellement sous forme solide est elle même caractérisée en fonction de la profondeur par des méthodes globales comme les dosages élémentaires en C, H, O, N et S. Néanmoins les molécules de carbone organique libres associées à cette matière organique peuvent être extraites avec des solvants appropriés afin d'en déterminer leur quantité et d'en réaliser l'analyse moléculaire par spectrométrie de masse.

Toutes ces études ont fait l'objet de nombreux articles publiées dans la littérature. Néanmoins les méthodes connues visent à décrire la biodégradation et non à la prédire. En effet, il est toujours proposé une équation globale de dégradation de la matière organique de type :

$$\text{matière organique} \Rightarrow CO_2 + 4H_2 \Rightarrow CH_4 + 2H_2O$$

Ce modèle ne tient pas compte de l'évolution structurale de la matière organique et admet que la totalité de celle ci peut être dégradée. Or, il est bien connu que l'efficacité de la dégradation de cette matière organique dépend des structures carbonées présentes dans cette dernière. Ainsi il est attendu que suivant la structure chimique de la matière organique présente, les vitesses et les quantités de gaz biogénique générés seront variables. S'il n'en était pas ainsi, il n'y aurait pas persistance de carbone organique à des profondeurs supérieures à 2500 m et donc pas de kérogène à l'origine de la formation du pétrole et du gaz.

Ainsi l'objet de l'invention concerne un procédé pour évaluer la quantité de gaz biogénique généré par biodégradation de matière organique dans une série sédimentaire d'un bassin sédimentaire. Le procédé se base sur une analyse des motifs structuraux de formules CxHyOvSzNw avec x>6, y>x, v <6, z<6, w<6, ainsi que sur un schéma réactionnel dans lequel ces motifs structuraux réagissent parallèlement avec des accepteurs d'électrons, et séquentiellement avec des accepteurs d'électrons.

Le procédé selon l'invention fournit un outil d'évaluation très utile notamment aux géologues soucieux d'orienter les investigations de nouvelles sources d'énergie fossile. En particulier, on peut l'utiliser dans le domaine technique de la modélisation de bassin pour prédire la quantité et la qualité du gaz hydrocarbure que l'on peut s'attendre à trouver dans les sédiments peu profonds, gaz lié à une altération microbienne de la matière organique sédimentaire en conditions de méthanogénèse.

## La méthode selon l'invention

De façon générale, l'invention concerne un procédé pour déterminer une quantité de gaz biogénique généré par biodégradation de matière organique dans une série sédimentaire d'un bassin sédimentaire. Il comporte les étapes suivantes :

i. on considère des motifs structuraux bioréactifs de formules CxHyOvSzNw avec x>6, y>x, v <6, z<6, w<6 ;

ii. on prélève un échantillon d'une roche de la série sédimentaire à une profondeur proche de la surface, telle que ladite roche n'a subi aucune biodégradation, et l'on détermine des quantités de chaque motif structural bioréactif présent dans ledit échantillon ;

iii. on définit un schéma réactionnel dans lequel lesdits motifs structuraux bioréactifs réagissent parallèlement avec des accepteurs d'électrons, et chacun des motifs structuraux bioréactifs réagit séquentiellement avec lesdits accepteurs d'électrons selon des vitesses de réactions individuelles pour chaque motif structural bioréactif, lesdites vitesses étant déterminées par des mesures expérimentales ;

iv. on détermine la quantité de gaz biogénique produit par biodégradation desdits motifs structuraux bioréactifs, au moyen dudit schéma réactionnel et desdites quantités de chaque motif structural présent dans ledit échantillon.

Selon l'invention, on peut déterminer les vitesses de réactions individuelles en réalisant les étapes suivantes :

a. on prélève au sein d'une série sédimentaire d'un bassin sédimentaire des échantillons de roche en surface et à différentes profondeurs ;

b. on détermine une proportion massique de chaque motif structural bioréactif au sein de chacun desdits échantillons au moyen d'extraction par un solvant organique puis d'une pyrolyse ;

c. on détermine une quantité de gaz biogénique produit par chaque motif structural bioréactif au moyen dudit schéma réactionnel et des proportions massiques ;

d. on en déduit les vitesses de réactions individuelles de chaque motif structural bioréactif.

Selon l'invention on peut regrouper les motifs structuraux bioréactifs en groupes structuraux bioréactifs. Enfin, le gaz biogénique peut être du méthane, et les accepteurs d'électrons peuvent être choisis parmi les accepteurs suivants : 02, N03, S04 et H2O.

D'autres caractéristiques et avantages de la méthode selon l'invention, apparaîtront à la lecture de la description ci-après d'exemples non limitatifs de réalisations, en se référant aux figures annexées et décrites ci-après.

La figure 1 illustre l'évolution de la concentration en sulfates (a) et en méthane (b) des eaux interstitielles (d'après Wefer *et al.,* 1998) ; le niveau en gris représente la zone de transition sulfate-méthane (ZTSM).

La figure 2 illustre l'ensemble de la démarche expérimentale pour caractériser et quantifier les motifs structuraux bioréactifs et réfractaires dans la matière organique solide et l'extrait associé.

Le procédé selon l'invention permet d'estimer la quantité de gaz biogénique généré par biodégradation de matière organique dans une roche d'une série sédimentaire au cours des processus biologiques successifs : aérobie, sulfato réduction et méthanogénèse. Pour cela, on évalue l'évolution de la structure chimique de la matière organique en réalisant un bilan de motifs structuraux bioréactifs qui disparaissent au cours des processus biologiques et en particulier au cours de la méthanogénèse. En effet, la matière organique sédimentaire est une macromolécule complexe de très haut poids moléculaire qui comprend à la fois des bio polymères issus d'organismes vivants; ainsi que des lipides de structures chimiques variées reliées entre elles par des groupements fonctionnels.

Le procédé comporte principalement les étapes suivantes:

1. on définit les motifs structuraux bioréactifs de la matière organique;

2. on réalise une estimation compositionnelle des motifs structuraux bioréactifs avant biodégradation ;

3. on définit un schéma réactionnel de biodégradation des motifs structuraux bioréactifs;

4. on évalue la quantité de gaz biogénique produit par biodégradation des motifs structuraux bioréactifs au moyen du schéma réactionnel et des quantités.

1- Motifs structuraux bioréactifs

L'action microbienne, à l'origine de la biodégradation de la matière organique sédimentaire, génère des gaz non hydrocarbures ($CO_2$, $H_2S$) et du méthane ($CH_4$) dit "gaz biogénique". Elle altère de façon séquentielle les structures oxygénées associées ou non à du soufre et de l'azote contenues dans la matière organique de départ.

On appelle motifs structuraux bioréactifs les structures chimiques de la matière organique initiale qui sont susceptibles d'être biodégradées.

Selon l'invention, on considère les motifs structuraux de formules CxHyOvSzNw avec x>6, y>x, v <6, z<6, w<6, pour décrire l'ensemble des motifs structuraux bioréactifs :

| structure chimique des motifs structuraux bioréactifs CxHyOvSzNw:x>6, y>x, v<6, z<6, w<6 | Fonctions chimiques | | | | | | | | | | | |
|---|---|---|---|---|---|---|---|---|---|---|---|---|
| | acides | | | | alcools | | | | cétones | | | |
| | mono | di | tri | poly | mono | di | tri | polv | mono | di | tri | poly |
| linéaires | x | x | x | x | x | x | x | x | x | x | x | x |
| linéaires ramifiés | x | x | x | x | x | x | x | x | x | x | x | x |
| mono cyclique saturés | x | x | x | x | x | x | x | x | x | x | x | x |
| di cyclique saturés | x | x | x | x | x | x | x | x | x | x | x | x |
| tri cyclique saturés | x | x | x | x | x | x | x | x | x | x | x | x |
| tetra cyclique saturés | x | x | x | x | x | x | x | x | x | x | x | x |

(suite)

| structure chimique des motifs structuraux bioréactifs | Fonctions chimiques | | | | | | | | | | | |
| | acides | | | | alcools | | | | cétones | | | |
| CxHyOvSzNw:x>6, y>x, v<6, z<6, w<6 | mono | di | tri | poly | mono | di | tri | polv | mono | di | tri | poly |
| penta cyclique saturés | x | x | x | x | x | x | x | x | x | x | x | x |
| poly cycliques saturés avec N > 5 | x | x | x | x | x | x | x | x | x | x | x | x |
| mono cyclique aromatiques | x | x | x | x | x | x | x | x | x | x | x | x |
| di cyclique aromatiques | x | x | x | x | x | x | x | x | x | x | x | x |
| tri cyclique aromatiques | x | x | x | x | x | x | x | x | x | x | x | x |
| tetra cyclique aromatiques | x | x | x | x | x | x | x | x | x | x | x | x |
| penta cyclique aromatiques | x | x | x | x | x | x | x | x | x | x | x | x |
| poly cycliques aromatiques avec N > 5 | x | x | x | x | x | x | x | x | x | x | x | x |

Tous ces motifs structuraux bioréactifs constituent la partie bio réactive de la matière organique initiale, appelée "matière organique bio réactive". Ces structures peuvent être regroupées pour des questions de simplification de calcul.

2- Estimation compositionnelle des motifs structuraux bioréactifs avant biodégradation

Pour quantifier la production de gaz biogénique, il est nécessaire de déterminer et quantifier les motifs structuraux bioréactifs et non bioréactifs dans les extraits de roche et dans la matière organique de départ (avant biodégradation). Les dosages d'oxygène dissous dans l'eau, ceux d'H2S et de méthane en fonction de la profondeur des sédiments permettent d'établir les intervalles de profondeur dans lesquels les réactions biologiques ont lieu. En particulier, le début de la zone de la méthanogénèse est défini par la disparition presque totale de l'H2S. La matière organique prélevée dans cette zone est donc considérée comme la matière organique initiale avant la méthanogénèse, processus à l'origine du gaz biogénique. Pour ce faire, on réalise les étapes suivantes :

- on isole dans une série sédimentaire une roche située en condition superficielle c'est à dire à moins de 10 m de profondeur ;

- on détruit la matrice minérale afin de concentrer la matière organique en utilisant par exemple la méthode de Durand et Nicaise (1980) ;

- on extrait les composés organiques solubles avec des solvants organiques comme par exemple le dichlorométhane. Ils constituent l'extrait de roche;

- on quantifie l'extrait récupéré par pesée ;

- on identifie les motifs structuraux par des méthodes appropriées et on les quantifie ;

- on réalise une pyrolyse douce c'est à dire en conditions anoxiques, sur la matière organique extraite ;

- on récupère les motifs structuraux libérés au cours de la pyrolyse douce par extraction avec des solvants organiques ;

- on quantifie les motifs structuraux libérés au cours de la pyrolyse par pesée;

- on caractérise les motifs structuraux libérés au cours de la pyrolyse par des méthodes appropriées ;

- on dresse ainsi une liste de pourcentage des différents motifs structuraux présents dans l'extrait et dans le pyrolysat.

Pour les extraits de roche, la détermination des motifs structuraux bioréactifs dans les hydrocarbures saturés et aromatiques sont analysés par exemple au moyen de la technique GC 2D, et les structures contenant des hétéro éléments pour être analysés par exemple par spectrométrie de masse haute résolution à transformée de Fourier ou par exemple par transformation en équivalents hydrocarbures. Pour la matière organique solide, il n'est pas possible d'en caractériser directement les motifs structuraux. Selon l'invention, on chauffe la matière organique sous atmosphère inerte à une température inférieure à 225°C pendant des durées de 3h à 24h pour avoir des rendements suffisants. Ainsi on libère des molécules organiques variées constitutives de la matière organique. Les hydrocarbures saturés et aromatiques peuvent être analysés par des méthodes comme par exemple la GC 2D et les structures contenant des hétéro éléments peuvent être analysées par exemple par spectrométrie de masse haute résolution à transformée de Fourrer ou par exemple par transformation en équivalents hydrocarbures. Cette pyrolyse libère les motifs structuraux portant les fonctions oxygénées, soufrées et azotées sans altérer ces derniers. Ainsi, en faisant les mêmes pyrolyses et les mêmes analyses sur des matières organiques biodégradées, on détermine par différence, la quantité et la qualité des motifs structuraux qui ont été détruits au cours d'altération microbienne et qui sont donc bioréactifs. On peut également déterminer par cette méthode la quantité et la qualité des motifs structuraux qui seraient au contraire produits par cette même altération.

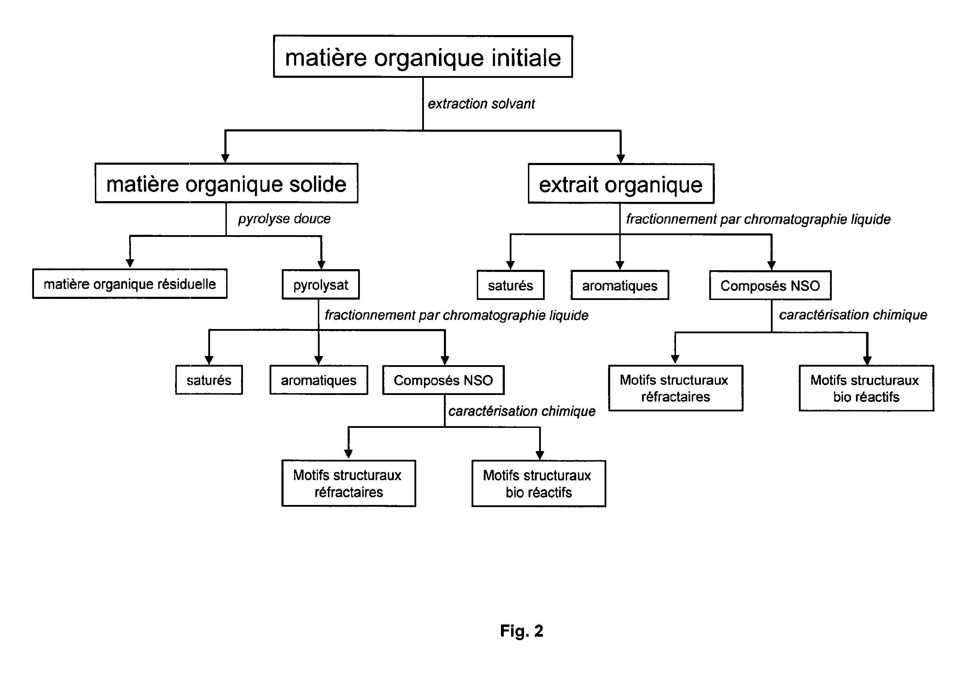

L'ensemble de ce procédé expérimental est décrit sur la figure 2.

3- Schéma compositionnel des motifs structuraux bioréactifs

Selon l'invention, le schéma réactionnel de biodégradation peut comporter un ensemble de réactions séquentielles pour chaque motif structural bioréactif contenu dans la matière organique de départ (extrait et matière organique solide) et qui aurait disparu au cours des différents processus d'altération. Les équations séquentielles de biodégradation des motifs structuraux bioréactifs avec les principaux accepteurs d'électrons ($O_2$, $NO_3$, $SO_4$ et $H_2O$), sont présentées ci-après.

**Accepteur d'électrons : $O_2$, biodégradation aérobie**

$$C_xH_yO_zS_vN_w + \left(x + \frac{1}{4}y - \frac{1}{2}z + 2v + \frac{2}{3}w\right)O_2 \rightarrow xCO_2 + \left(\frac{1}{2}y\right)H_2O + vSO_4 + wNO_3 \qquad \textbf{eq. 1}$$

**accepteur d'électrons : $NO_3$, dénitrification**

$$C_xH_yO_zS_vN_w + \frac{1}{3}\left(2x + \frac{1}{2}y - z + 4v\right)NO_3^- \rightarrow xCO_2 + \frac{1}{2}yH_2O + vSO_4 + \left[\frac{1}{6}\left(2x + \frac{1}{2}y - y + 4v\right) + \frac{1}{2}w\right]N_2 \qquad \textbf{eq. 2}$$

**accepteur d'électrons : $SO_4$, sulfatoréduction**

$$C_xH_yO_zS_vN_w + \frac{1}{5}\left(2x + \frac{1}{2}y - z - v\right)SO_4 \rightarrow xCO_2 + \frac{1}{5}(2y - 2x + z - 4v)H_2O + \frac{1}{5}\left(2x + \frac{1}{2}y - z + 4v\right)H_2S + \frac{1}{2}wN_2 \qquad \textbf{eq. 3}$$

**accepteur d'électrons : H$_2$O, méthanogénèse**

$$C_xH_yO_zS_vN_w + (x-z)H_2O \rightarrow \frac{1}{2}xCH_3CO_2^- + \frac{1}{2}xH^+ + \frac{1}{2}(y-z-v)H_2 + vH_2S + \frac{1}{2}wN_2 \qquad \textbf{eq. 4}$$

$$CH_3CO_2^- + H^+ \rightarrow CH_4 + CO2 \text{ eq. 5}$$

$$CO_2 + 4H_2 \rightarrow CH_4 + 2H2O \text{ eq. 6}$$

$$C_xH_yO_zS_vN_w + \left(x-\frac{1}{4}y-\frac{1}{2}y+\frac{1}{2}v\right)H_2O \rightarrow \frac{1}{2}\left(x-\frac{1}{4}y+\frac{1}{2}z+\frac{1}{2}v\right)CO_2 + \frac{1}{2}\left(x+\frac{1}{4}y-\frac{1}{2}z-\frac{1}{2}v\right)CH_4 + vH_2S + \frac{1}{2}wN_2$$

$$\textbf{eq. 7}$$

Ainsi, le schéma réactionnel de biodégradation selon l'invention est défini par l'ensemble des réactions chimiques suivantes :

- pour chaque motif structural bioréactif, les réactions avec les accepteurs d'électrons (oxygène, nitrate, sulfate ou H$_2$O) sont séquentielles : chaque motif structural réagit avec l'accepteur d'électrons ayant le potentiel d'oxydoréduction le plus fort, jusqu'à épuisement dans l'eau de cet accepteur, puis réagit avec l'accepteur d'électrons restant dans le milieu et ayant le potentiel d'oxydoréduction le plus fort. Ce mécanisme peut se répéter jusqu'à épuisement soit des accepteurs d'électrons, soit de la matière organique bio réactive.

- les motifs structuraux bioréactifs réagissent en parallèle : il n'existe pas de mécanisme séquentiel, dans lequel un motif structural bioréactif réagirait une fois qu'un autre motif structural bioréactif ait été complètement dégradé. En fait, tous les motifs structuraux bioréactifs peuvent réagir en même temps, mais avec des cinétiques différentes, définies par des vitesses de réactions individuelles (Vi).

L'identification et la quantification des motifs structuraux bioréactifs initiaux (étape 2 du procédé) permet de connaître les coefficients x, y, z, v et w des équations 1, 2, 3 et 7 du schéma réactionnel. Pour définir complètement ce schéma réactionnel, il faut définir les différentes vitesses de réactions individuelles (Vi) pour les réactions séquentielles.

*Détermination des vitesses de réactions individuelles (Vi) par évaluation des pertes relatives des motifs structuraux bioréactifs*

La détermination des pertes des différents motifs structuraux bioréactifs est réalisée à la fois sur l'analyse des extraits de roche et ceux obtenus par pyrolyse douce de la matière organique solide. Ces deux analyses sont faites sur des échantillons prélevés à des profondeurs croissantes. La matière organique déposée dans les sédiments est échantillonnée en fonction de la profondeur généralement entre 0 et 1500 m, mais des échantillons peuvent être prélevés jusqu'à des profondeurs de 2500 m. Ainsi, une série de matière organique est récupérée à des profondeurs croissantes d'enfouissement. L'analyse de ces échantillons est la même qu'à l'étape 2 du procédé selon l'invention.

La décroissance des quantités de motifs structuraux bioréactifs en fonction de la profondeur correspond donc aux pertes associées aux activités bactériennes. Ainsi sur une série de matières organiques prélevées à différentes profondeur, on détermine les pertes pour chacun des motifs structuraux bioréactifs entre deux profondeurs données. La somme de ces pertes constitue la perte totale de la matière organique de départ à un niveau donné d'altération biologique. Ainsi, on calcule les pertes en motifs structuraux pour chacune des zones de profondeurs correspondant à une réaction biologique donnée.

Connaissant les pertes relatives des différents motifs structuraux bioréactifs en fonction de l'avancement de la réaction biologique, on détermine les différentes vitesses de réactions individuelles (Vi) pour les réactions séquentielles.

On peut par exemple utiliser la méthode décrite dans la demande de brevet EP 2 154 254. On classe les motifs structuraux bioréactifs par ordre décroissant de perte en fonction de la profondeur. On attribue alors des coefficients dont la valeur la plus grande est attribuée au motif structural bioréactif le plus sensible. Puis, on optimise ces coefficients en comparant les proportions relatives obtenues entre les motifs structuraux avec celles mesurées sur les échantillons naturels en fonction de la profondeur. Ainsi, en sommant ces coefficients optimisés et en les normalisant à 100%, on obtient des vitesses de réactions individuelles (Vi) pour les réactions séquentielles.

4- Détermination des volumes de gaz biogéniques générés

Chaque équation (1, 2, 3 et 7) est appliquée sur chaque motif structural bioréactif. Parmi ces équations, l'équation 7 décrit l'ensemble du processus de formation du gaz biogénique (CH$_4$). Ainsi, connaissant le taux de perte de chacun des motifs structuraux, on calcule en utilisant l'équation 7 sur chacun des motifs structuraux bioréactifs, les quantités de méthane généré. En sommant ces quantités de méthane généré à partir de chacun des motifs structuraux bioréactifs, on calcule la quantité totale de méthane produite.

**Exemple**

On considère une masse de 1000 kg de sédiments qui contient 4% de matière organique soit 40 kg de matière organique. Si cette matière organique au début de la méthanogénèse contient 10% du motif structural bioréactif C40H8002 (poids moléculaire égal à 592 g), cela correspond à une masse de 4 kg d'acides. Si 10 % de ce motif structural bioréactif sont dégradés, cela correspond à une perte de 400 grammes soit 0.675 mole. En appliquant l'équation 7, la dégradation microbienne de 0.675 mole de C40H8002 correspond à la production de 19.9 moles ou 446 litres de CH4 pour 1 tonne de sédiment.

**Revendications**

1. Procédé pour déterminer une quantité de gaz biogénique généré par biodégradation de matière organique dans une série sédimentaire d'un bassin sédimentaire, **caractérisé en ce que** l'on réalise les étapes suivantes :

    i. on considère des motifs structuraux bioréactifs de formules CxHyOvSzNw avec x>6, y>x, v <6, z<6, w<6 ;
    ii. on prélève un échantillon d'une roche de la série sédimentaire à une profondeur proche de la surface, telle que ladite roche n'a subi aucune biodégradation, et l'on détermine des quantités de chaque motif structural bioréactif présent dans ledit échantillon ;
    iii. on définit un schéma réactionnel dans lequel lesdits motifs structuraux bioréactifs réagissent parallèlement avec des accepteurs d'électrons, et chacun des motifs structuraux bioréactifs réagit séquentiellement avec lesdits accepteurs d'électrons selon des vitesses de réactions individuelles pour chaque motif structural bioréactif, lesdites vitesses étant déterminées par des mesures expérimentales ;
    iv. on détermine la quantité de gaz biogénique produit par biodégradation desdits motifs structuraux bioréactifs, au moyen dudit schéma réactionnel et desdites quantités de chaque motif structural présent dans ledit échantillon.

2. Procédé selon la revendication 1, dans lequel on détermine les vitesses de réactions individuelles en réalisant les étapes suivantes :

    a. on prélève au sein d'une série sédimentaire d'un bassin sédimentaire des échantillons de roche en surface et à différentes profondeurs ;
    b. on détermine une proportion massique de chaque motif structural bioréactif au sein de chacun desdits échantillons au moyen d'extraction par un solvant organique puis d'une pyrolyse ;
    c. on détermine une quantité de gaz biogénique produit par chaque motif structural bioréactif au moyen dudit schéma réactionnel et des proportions massiques;
    d. on en déduit les vitesses de réactions individuelles de chaque motif structural bioréactif.

3. Procédé selon l'une des revendications précédentes, dans lequel on regroupe lesdits motifs structuraux bioréactifs en groupes structuraux bioréactifs.

4. Procédé selon l'une des revendications précédentes, dans lequel ledit gaz biogénique est du méthane.

5. Procédé selon l'une des revendications précédentes, dans lequel lesdits accepteurs d'électrons sont choisis parmi les accepteurs suivants : O$_2$, NO$_3$, SO$_4$ et H$_2$O.

**Fig. 1**

```
                        matière organique initiale

                                    │
                            extraction solvant
                                    │
              ┌─────────────────────┴─────────────────────┐
              ▼                                            ▼
     matière organique solide                      extrait organique

              │                                            │
        pyrolyse douce              fractionnement par chromatographie liquide
        ┌─────┴──────┐              ┌──────────────┼──────────────┐
        ▼            ▼              ▼              ▼              ▼
matière organique  pyrolysat     saturés      aromatiques    Composés NSO
  résiduelle
                       │                                            │
        fractionnement par chromatographie liquide        caractérisation chimique
        ┌──────────┼──────────┐                           ┌──────────┴──────────┐
        ▼          ▼          ▼                           ▼                     ▼
     saturés  aromatiques  Composés NSO            Motifs structuraux    Motifs structuraux
                                                     réfractaires          bio réactifs
                              │
                    caractérisation chimique
                    ┌─────────┴─────────┐
                    ▼                   ▼
            Motifs structuraux   Motifs structuraux
              réfractaires         bio réactifs
```

**Fig. 2**

Europäisches
Patentamt

European
Patent Office

Office européen
des brevets

**RAPPORT DE RECHERCHE EUROPEENNE**

Numéro de la demande

EP 12 29 0141

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 2 154 254 A2 (INST FRANCAIS DU PETROLE [FR] IFP ENERGIES NOUVELLES [FR]) 17 février 2010 (2010-02-17) * alinéa [0006] * * alinéas [0014] - [0022] * * alinéas [0026] - [0030] * * alinéas [0032], [0033] * ----- | 1-5 | INV. C09K8/00 C12Q1/64 G01N33/24 |
| A | WO 2007/118094 A2 (LUCA TECHNOLOGIES LLC [US]; PFEIFFER ROBERT S [US]; ULRICH GLENN A [US) 18 octobre 2007 (2007-10-18) * alinéa [0030] * ----- | 1-5 | |
| A | WO 02/34931 A2 (GUYER JOE E [US]; SCOTT ANDREW R [US]) 2 mai 2002 (2002-05-02) * page 10, alinéa 2 * * page 12, dernier alinéa - page 14, alinéa 1 * ----- | 1-5 | |

DOMAINES TECHNIQUES
RECHERCHES (IPC)

C09K
C12Q
E21B
G01N

Le présent rapport a été établi pour toutes les revendications

| Lieu de la recherche | Date d'achèvement de la recherche | Examinateur |
|---|---|---|
| Munich | 23 août 2012 | Michalitsch, Richard |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 12 29 0141

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-08-2012

| Document brevet cité au rapport de recherche | | Date de publication | Membre(s) de la famille de brevet(s) | | Date de publication |
|---|---|---|---|---|---|
| EP 2154254 | A2 | 17-02-2010 | EP | 2154254 A2 | 17-02-2010 |
| | | | FR | 2934605 A1 | 05-02-2010 |
| | | | US | 2010076735 A1 | 25-03-2010 |
| WO 2007118094 | A2 | 18-10-2007 | AU | 2007234895 A1 | 18-10-2007 |
| | | | CA | 2648752 A1 | 18-10-2007 |
| | | | EP | 2010753 A2 | 07-01-2009 |
| | | | NZ | 572426 A | 25-05-2012 |
| | | | US | 2007261843 A1 | 15-11-2007 |
| | | | US | 2010190203 A1 | 29-07-2010 |
| | | | WO | 2007118094 A2 | 18-10-2007 |
| WO 0234931 | A2 | 02-05-2002 | AU | 2444502 A | 06-05-2002 |
| | | | US | 2004033557 A1 | 19-02-2004 |
| | | | WO | 0234931 A2 | 02-05-2002 |

EPO FORM P0460

Pour tout renseignement concernant cette annexe : voir Journal Officiel de l'Office européen des brevets, No.12/82

RÉFÉRENCES CITÉES DANS LA DESCRIPTION

**Documents brevets cités dans la description**

• EP 2154254 A